Europäisches Patentamt

European Patent Office

Office européen des brevets

(11) Numéro de publication: **0 433 275 B1**

## (12) FASCICULE DE BREVET EUROPEEN

(45) Date de publication de fascicule du brevet: **29.12.93**

(51) Int. Cl.5: **A61K 33/14**, A61K 31/725,
//(A61K33/14,31:725,31:70)

(21) Numéro de dépôt: **88905877.2**

(22) Date de dépôt: **17.06.88**

(86) Numéro de dépôt internationale :
**PCT/FR88/00326**

(87) Numéro de publication internationale :
**WO 88/10117 (29.12.88 88/28)**

(54) **COMPOSITION A BASE DE MUCOPOLYSACCHARIDES HEPARINIOUES, ACTIVE PAR VOIE ORALE.**

(30) Priorité: **19.06.87 FR 8708670**

(43) Date de publication de la demande:
**26.06.91 Bulletin 91/26**

(45) Mention de la délivrance du brevet:
**29.12.93 Bulletin 93/52**

(84) Etats contractants désignés:
**AT BE CH DE GB IT LI LU NL SE**

(56) Documents cités:
**WO-A-85/05362**
**FR-M- 37 16M**

(73) Titulaire: **OPOCRIN S.p.A.**
**Via Pacinotti, 3**
**I-41040 Corlo (Mo)(IT)**

(72) Inventeur: **OPOCRIN S.p.A.**
**Via Pacinotti, 3**
**I-41040 Corlo (Mo)(IT)**

(74) Mandataire: **Gutmann, Ernest et al**
**S.C. Ernest Gutmann - Yves Plasseraud**
**67, boulevard Haussmann**
**F-75008 Paris (FR)**

## Description

L'invention concerne une composition à base de mucopolysaccharides hépariniques possédant des propriétés régulatrices vis-à-vis de la coagulation sanguine, lorsqu'elle est administrée par voie orale.

L'expression "mucopolysaccharides hépariniques" (ou l'expression équivalente "glycosaminoglycanes", telle qu'elle est utilisée dans la présente description, recouvre tous les mucopolysaccharides capables d'agir sur au moins l'un des facteurs de la coagulation sanguine, parmi lesquels, notamment :

- l'héparine que l'on pourrait qualifier de "classique" formée de chaînes polysaccharidiques dont les poids moléculaires s'étagent normalement d'environ 6000 à 50.000,
- des compositions ou mélanges formés de chaînes polysaccharidiques plus courtes, en particulier des oligosaccharides pouvant comporter de 5 à 30 motifs saccharidiques, notamment des mélanges obtenus par fragmentation physique ou chimique de chaînes d'héparine plus longues,
- des oligosaccharides de synthèse comportant notamment de 5 à 8 motifs saccharidiques, plus particulièrement ceux qui sont porteurs d'un site de fixation à l'AT III,
- des sels des divers mucopolysaccharides hépariniques sus-mentionnés, notamment les sels de sodium, potassium, calcium, magnésium.

Les possibilités thérapeutiques qu'offrent les mucopolysaccharides hépariniques dans des thérapies mettant en jeu leurs capacités d'agir sur un ou plusieurs facteurs intervenant au niveau de la coagulation sanguine sont bien connues. Il n'est pas besoin d'insister sur les propriétés anticoagulantes bien connues de l'héparine elle-même. L'efficacité des mucopolysaccharides hépariniques de poids moléculaires plus faibles, plus particulièrement ceux qui se caractérisent par une affinité pour l'AT III, a été démontrée. Ils sont utilisables avec succès pour la prévention ou le traitement de thromboses veineuses ou artérielles ou d'autres manifestations thrombo-emboliques veineuses ou artérielles.

Plusieurs protocoles d'administration de ces compositions de mucopolysaccharides hépariniques, notamment par voie intraveineuse, intraartérielle ou sous cutanée sont bien au point. Ces modes d'aministrations tous par voie parentérale, sont cependant source d'inconfort et de troubles (hématomes...) pour les patients et d'autant plus contraignants que beaucoup de traitements peuvent requérir plusieurs injections quotidiennes, pendant des périodes prolongées.

Certes d'autres voies d'administration que la voie parentérale ont été proposées. On citera par exemple, les modes d'administration par la voie rectale (grâce à l'utilisation de suppositoires), la voie cutanée (par l'intermédiaire de pommades) la voie pulmonaire (au moyen d'inhalations), la voie sub-linguale, etc... L'efficacité des compositions utilisées dans ces nouvelles voies d'administration demeure toutefois souvent locale, lente et minime, voire aléatoire.

Cependant, malgré les nombreuses recherches effectuées jusqu'a ce jour, il n'a pas encore été possible de mette au point des compositions à base de mucopolysaccharides hépariniques, réellement efficaces lorsqu'elles sont administrées par voie orale, et ce malgré la stabilité connue de l'héparine en milieu gastrique.

La demande PCT 85/05362 décrit des compositions à base de multiplets hépariniques ou complexes, susceptibles d'entrer dans la préparation de compositions administrables par voie orale. Ces multiplets sont obtenus par réaction de l'héparine sous forme d'acide et d'un cation ammonium quaternaire.

L'invention a pour but de remédier à cette difficulté majeure, plus particulièrement de fournir des compositions de mucopolysaccharides hépariniques susceptibles, lorsqu'elles sont administrées par voie orale, de manifester leurs propriétés régulatrices de la coagulation sanguine in vivo avec un niveau d'efficacité suffisant pour permettre leur utilisation en thérapeutique sous cette forme d'administration pour le traitement des mêmes types d'affections que celles qui ne peuvent à ce jour être traités que par des compositions de mucopolysaccharides hépariniques administrées par voie parentérale.

La composition selon l'invention est caractérisée en ce qu'elle contient en combinaison :

a/ au moins un composé mucopolysaccharide héparinique,

b/ au moins un ose physiologiquement acceptable, capable d'être absorbé au niveau de la muqueuse intestinale,

c/ au moins un sel ionique physiologiquement acceptable d'un cation métallique, les constituants a/, b/, c/ étant présents en proportions mutuelles permettant l'induction par ladite composition d'un effet anticoagulant in vivo lorsqu'elle est administrée par voie orale.

Les inventeurs ont en effet découvert que l'association dans une composition, des constituants b/ et c/ précités aux mucopolysaccharides a/ favorisait le franchissement par ces derniers de la muqueuse digestive et leur passage dans la circulation sanguine. Il ne s'agit là bien entendu que d'une hypothèse qui ne saurait être considérée comme un mode d'explication définitif des modes d'action respectifs des divers constituants essentiels de la composition selon l'invention.

EP 0 433 275 B1

Dans une composition préférée selon l'invention, l'ose est un hexose tel que le glucose ou le lévulose.

De préférence encore, le sel de cation métallique entrant dans la composition selon l'invention est un chlorure. Un des intérêts particuliers de ce choix résulte de la bonne compatibilité des chlorures avec les fonctions métaboliques. Le cation métallique du chlorure appartient avantageusement à la famille des métaux alcalinoterreux, notamment calcium ou magnésium, ou à la famille des métaux alcalins, notamment sodium ou potassium. Le plus souvent ce sel sera un chlorure de sodium ou de préférence de calcium.

Il est à noter que lorsque l'héparine est à l'état de sel de calcium (héparinate de calcium), celui-ci n'intervient guère dans l'activité par voie orale de la composition En effet, un mélange d'héparinate de calcium et de glucose, dépourvu d'un sel ionique d'un cation distinct, n'a pas d'efficacité significative par voie orale.

De même il a été constaté que des compositions d'héparine et de sels ioniques, mais dépourvues de glucose, n'ont pas d'efficacité significative en vue d'un usage thérapeutique lors de l'administration par voie orale.

Il résulte déjà de ce qui précède que les trois constituants essentiels de la composition doivent être dans des proportions mutuelles respectivement déterminées pour assurer l'efficacité de la composition, lorsque celle-ci est administrée par voie orale.

Il est entendu que les exemples de compositions qui suivent ne limitent en rien le choix des constituants susceptibles d'être associés dans la composition active par voie orale, choix qui pourra éventuellement entraîner aussi des variations au niveau des proportions optimales des trois constituants éventuellement distincts (surtout au niveau des oses et des sels ioniques).

Ceci étant, des compositions préférées efficaces par voie orale comprennent des proportions relatives suivantes des composants mucopolysaccharides héparinique, ose, sel de cation :
- de 600 à 2500 unités USP selon le cas, ou de 600 à 2500 unités anti-Xa ou de 4 à 17mg de mucopolysaccharide héparinique
- de 4 à 60mg d'ose
- de 0,5 à 100mg de sel ionique de cation

Des compositions préférées particulières comprennent notamment :
- de 600 à 2500 unités USP selon le cas, ou de 600 à 2500 unités anti-Xa ou de 4 à 17 mg de mucopolysaccharide héparinique
- de 4 à 50mg de glucose,
- de 10 à 80mg de chlorure de calcium correspondant à une proportion de 1,83mg à 14,64mg d'ion calcium et de 3,24mg à 19,92mg d'ion chlore

Une deuxième composition efficace comprend :
- de 600 à 2500 unités USP selon le cas, ou de 600 à 2500 unités anti-Xa ou de 4 à 17mg de mucopolysaccharide héparinique
- de 4 à 60mg de lévulose ou d-fructopyranose
- de 10 à 80mg de chlorure de calcium

Une autre composition efficace comprend :
- de 600 à 2500 unités USP selon le cas, ou de 600 à 2500 unités anti-Xa ou de 4 à 17mg de mucopolysaccharide héparinique
- de 5 à 50mg de glucose et
- de 0,9 à 9mg de chlorure de sodium,

Les unités anti-Xa sont exprimées en prenant pour référence l'étalon international d'héparine pour les activités plasmatiques.

Les compositions pharmaceutiques selon l'invention pour l'administration par voie orale, comprennent d'une façon générale les constituants tels que décrits précédemment dans les proportions relatives mutuelles qui ont été indiquées avec une dose efficace de mucopolysaccharide héparinique pour induire in vivo l'effet régulateur recherché vis-à-vis de la coagulation, le cas échéant, en association avec un véhicule pharmaceutique acceptable, approprié à l'administration par voie orale. En particulier les excipients solides ou liquides sont choisis selon la forme d'administration orale choisie : comprimés, gelules, solutés buvables etc...).

Un mode d'administration avantageux des compositions selon l'invention est l'administration sous forme de solution, dans un véhicule pharmaceutiquement acceptable.

Une présentation préférée des compositions selon l'invention consiste à placer cette composition dans un volume de 0,7 à 1ml de solution, notamment 0,8ml.

Les compositions selon l'invention, peuvent également être administrées par voie rectale, sublinguale ou toute autre voie efficace.

3

Une dose unitaire de cette composition pharmaceutique contient par exemple de 600 à 2500 unités USP d'héparine.

Une posologie d'administration chez l'homme est mentionnée à titre indicatif comme suit :

- La composition selon l'invention est administrée par voie orale à un patient de taille normale (60 à 80kg), sous forme de 1000 à 100.000 unités USP selon le cas, ou 1000 à 100.000 unités anti-Xa calculés par rapport à l'héparine, selon la pathologie à traiter ou à prévenir,
- L'administration est répétée à un intervalle de 3 à 4 fois par jour.

Des aménagements des modalités de ce traitement peuvent naturellement être envisagés afin de prendre en considération les résultats connus d'analyses effectuées avant et pendant le traitement.

Selon un autre aspect de l'invention, les compositions décrites ci-dessus sont applicables à la production de médicaments administrables par voie orale, ayant une activité régulatrice vis-à-vis de la coagulation sanguine.

D'autres caractéristiques des compositions selon l'invention apparaîtront encore au cours de la description qui suit, de compositions préférées conformes à l'invention, des études pharmacologiques qui ont été effectuées avec ces compositions et des résultats qui ont été obtenus. Ces résultats rendent compte de la possible efficacité des compositions selon l'invention, administrées par voie orale, pour la prévention et le traitement des différents désordres de la coagulation normalement traités par des compositions de mucopolysaccharides administrées par voie parentérale.

Avant d'exposer les détails de chacun des exemples illustrant l'invention, un protocole opératoire général est rappelé. Il est entendu que les modifications de ce protocole spécifiques aux différents exemples, seront signalées au cours de l'exposé.

Protocole Général appliqué dans la réalisation des exemples.

La composition est administrée à l'animal (lapin ou rat) par voie orale au temps t = 0, à l'aide d'une canule métallique coudée montée sur une seringue.

Afin d'effectuer les prélèvements de sang ultérieurs chez le lapin, un cathéter est posé dans la veine fémorale au niveau de la partie interne de la cuisse de chaque lapin après avoir fait une incision d'environ 2 centimètres afin de dégager la veine fémorale. Lorsque les animaux utilisés sont des rats les prélèvements de sang sont faits de façon intracardiaque. Les lapins sont ensuite placés sous perfusion de sérum physiologique dans une boite de contention alors que les rats sont sacrifiés.

Pour réaliser les tests biologiques, des prélèvements de sang sont effectués au temps t = 0, juste avant l'administration de l'héparine, puis aux temps t = 1 heure, t = 2 heures, t = 3 heures. Le sang est chaque fois recueilli dans des tubes de marque EPPENDORF, sur du citrate à 9%, puis centrifugé. Le plasma est ensuite séparé en deux tubes, dont l'un est congelé et conservé, l'autre permettant de réaliser les tests biologiques.

Les tests biologiques dont il est question dans l'invention sont des dosages plasmatiques destinés à mesurer l'absorption duodénale de la substance administrée, vérifiable par l'induction d'une activité plasmatique. Cette activité plasmatique relève de l'étude de 4 tests décrits comme suit :

1/ un test de coagulabilité globale, le temps de céphaline-kaolin (T.C.K.)

Le T.C.K. est un test qui permet l'étude de la voie endogène de la coagulation, donc l'étude des facteurs inhibés du fait de l'affinité de l'héparine pour l'antithrombine III (AT III). Ce test est réalisé à l'aide des réactifs commercialisés par les Laboratoires STAGO, en utilisant la méthode classique selon Langdell et al. (dans "Effect of anti-hemophilic Factor on one-stage clotting tests". J. Lab. Clin. Insert 41-637,647, 1953) et Larrieu H J, G. Weiland (dans "Utilisation de la Céphaline dans les tests de coagulation" Nouv. Rev. Française, Hématol, 12-2, 199-210, 1957).

2/ l'activité antithrombine-III

- le temps de thrombine (TT).

Le temps de thrombine permet d'étudier la capacité inhibitrice de l'héparine sur la thrombine, via l'antithrombine III. Le temps de thrombine mesuré est en réalité un temps de thrombine concentré et qui ne s'allonge qu'en présence d'héparine à forte concentration.

3/ Le temps de titrarine.

Ce temps de coagulation mesuré en présence de titrarine (Trombine prétitrée) est le temps de coagulation du plasma, obtenu directement en fonction de l'héparine circulante. Il permet donc un contrôle de la mesure du temps de thrombine.

4/ l'activité anti-Xa : HEPACLOT.

Ce test biologique est un test chronométrique qui permet l'étude de l'activité plasmatique anti-Xa de l'héparine selon la méthode mise au point par le Laboratoire STAGO. Ce test a été effectué avec les réactifs appropriés commercialisés par le Laboratoire STAGO.

Les essais qui suivent ont été réalisés le plus souvent avec une héparine commerciale. Il s'agit d'un sel de calcium de l'héparine, ayant un titre de 25.000 Unités/ml (ces unités étant désignées dans ce qui suit par la lettre "U"). Dans le cas où une autre héparine aura été utilisée mention expresse en sera faite.

Les résultats sont, le cas échéant, illustrés dans les dessins, à l'aide de représentations graphiques faisant apparaître ces résultats sous forme de barres verticales, dont les hauteurs correspondent à des temps mesurés, par référence à l'échelle et aux indications fournies sur les axes des ordonnées, dans les conditions exprimées dans les exemples correspondants et, quelquefois, sous les axes des abscisses.

EXEMPLE I

Administration par voie orale d'une composition contenant de l'héparine à différentes concentrations en présence de chlorure de calcium et de glucose.

Les animaux utilisés sont des lapins mâles adultes de race "ALBINOS" de souche néo-zélandaise, d'un poids moyen de 2 kg 500 provenant de l'élevage EVIC CEBA de Blanquefort (33290).

Ces lapins sont anesthésiés par administration intra-musculaire de l'anesthésique connu sous la marque commerciale KETALAR (100mg/kg)(Laboratoire LILLY).

La composition administrée par voie orale est une solution d'un mélange contenant 2500 unités USP ou 2500 unités anti Xa ou 15mg d'héparine calcique, 30mg de chlorure de calcium et 20mg de glucose pour 0,80ml de mélange.

Trois posologies ont été étudiées par voie orale. Trois groupes de lapins reçoivent des doses du susdit mélange, à concurrence de 1250, 600 et 300 U/kg de lapin respectivement.

A titre de comparaison, un autre groupe de lapins reçoit 1250 U d'héparine (par kg de lapin) par la voie sous-cutanée ; un groupe contrôle reçoit la même dose d'héparine par voie orale mais en l'absence des deux autres constituants, et un groupe placebo reçoit un même volume de solution physiologique.

Les dosages plasmatiques intéressants sont réalisés grâce aux tests biologiques T.C.K. et T.T. définis dans le protocole général.

Les résultats relatifs à ces essais sont résumés dans les tableaux I et II suivants :

TABLEAU I
Mesure des T.C.K.

| t mn | Voie sous-cut. 1250 U/Kg | Voie orale | | | Contrôle | Placebo |
|---|---|---|---|---|---|---|
| | | 1250 U/Kg | 600 U/Kg | 300 U/Kg | | |
| 0 | 26 ± 2 n = 3 | 25.5 ± 4.7 n = 7 | 22.3 ± 2.9 n = 5 | 24.7 ± 0.3 n = 2 | 18.5 | 18 |
| 10 | 26.5 ± 1.7 n = 2 | 25.8 ± 6.4 n = 6 | 23.9 ± 2.5 n = 5 | 24.5 ± 0.7 n = 2 | 17 | - |
| 20 | 36 ± 6.9 n = 3 | 26 ± 7.6 n = 5 | 25.8 ± 1.3 n = 5 | 25.5 ± 2.1 n = 2 | 18.5 | - |
| 30 | 40.3 ± 8 n = 3 | 31.1 ± 12 n = 6 | 28.3 ± 2.9 n = 5 | 24.7 ± 2.4 n = 2 | 22 | - |
| 40 | 41 ± 8.7 n = 3 | 39.4 ± 15 n = 5 | 28.1 ± 4.4 n = 5 | 24.2 ± 1.7 n = 2 | 21.5 | - |
| 50 | 44.6 ± 12 n = 3 | 43.5 ± 17 n = 7 | 29.7 ± 5.4 n = 4 | 24.5 ± 3.5 n = 2 | 21 | - |
| 60 | 49.8 ± 15 n = 3 | 52.2 ± 28 n = 7 | 31.6 ± 6 n = 5 | 25 ± 2.8 n = 2 | 21 | 18 |
| 120 | 66.6 ± 32 n = 3 | 64.4 ± 45 n = 7 | 43.5 ± 28 n = 5 | 24.7 ± 3.1 n = 2 | 20 | 18.5 |
| 180 | 75 ± 37 n = 3 | 69.7 ± 53 n = 7 | 39.4 ± 26 n = 5 | 27 ± 5.6 n = 2 | 22 | 19 |
| 240 | 76 ± 42 n = 3 | 63.1 ± 36 n = 7 | 44.2 ± 31 n = 5 | 28 ± 7 n = 2 | 21.5 | 18.5 |
| 300 | 90 | - | - | - | 26 | 19.5 |

6

## TABLEAU II
### Mesure des TT.

| t mn | Voie sous-cut. 1250 U/K → | Voie orale | | | Contrôle | Placebo |
|---|---|---|---|---|---|---|
| | | 1250 U/Kg → | 600 U/Kg → | 300 U/Kg → | | |
| 0 | 9.33 ± 2.75 n = 3 | 8.35 ± 0.85 n = 7 | 8 ± 1.41 n = 5 | 7.5 n = 2 | 8.5 | 7.5 |
| 10 | 9.66± 2.88 n = 3 | 8.6 ± 1.21 n = 6 | 8.1 ± 1.08 n = 5 | 7.75 ± 0.35 n = 2 | 8.5 | - |
| 20 | 11.66± 5.5 n = 3 | 17 ± 21 n = 6 | 8.4± 0.41 n = 5 | 7.5 n = 2 | 8.5 | - |
| 30 | 18.16± 10.39 n = 3 | 17.6± 20.7 n = 6 | 9.2 ± 1.68 n = 5 | 7.5 n = 2 | 9 | - |
| 40 | 28.33± 27.46 n = 3 | 17.6± 20 n = 6 | 10.1± 1.98 n = 4 | 7.75±0.35 n =2 | 8.5 | - |
| 50 | 44.16± 27.42 n = 3 | 23.5±24.4 n = 7 | 22.2± 25.2 n = 5 | 7.5 ± 0.7 n = 2 | 8.5 | - |
| 60 | 44.66± 26.55 n = 3 | 24.1±24.6 n = 7 | 23.3± 22.48 n = 5 | 7.7 ± 0.35 n = 2 | 9.5 | 7.5 |
| 120 | 46.66±23.09 n = 3 | 23.5±24.9 n = 7 | 29.4±27.9 n = 5 | 7.7 ± 0.7 n = 2 | 8.5 | 7.5 |
| 180 | 60 n = 3 | 24 ± 24.7 n = 7 | 19.7± 22.5 n = 5 | 7.5 n = 2 | 8 | 7.5 |
| 240 | 60 n = 3 | 24.1±24.5 n = 7 | 19.6± 22.6 n = 5 | 7.75±0.35 n = 2. | 9.5 | 7 |
| 300 | - | - | - | - | 8.5 | 7 |

L'examen des résultats contenus dans ces tableaux conduit à la conclusion que l'héparine est bien absorbée par voie orale chez le lapin en particulier à des doses dépassant 300 U/kg, par exemple à des doses de 600 U/kg et 1250 U/kg. A la dose de 1250 U/kg on observe avec le mélange constitué par la composition administrée par voie orale, des augmentations du temps de coagulation sanguine mesuré en TCK, qui sont du même ordre de grandeur que celles observées avec l'héparine administrée par voie sous-

EP 0 433 275 B1

cutanée. Les variations du temps TCK induits dans les groupes Contrôle et Placebo ne présentent eux-mêmes aucun caractère significatif.

Une étude similaire a été réalisée sur un groupe de 12 rats de souche "WISTAR", d'un poids moyen de 200g (élevage EVIC-CEBA).

L'héparine a été administrée à 3 dosages différents (Groupes 2, 3 et 4 de rats). L'étude est complétée par un groupe témoin (groupe 1).

Groupe 1 :     sérum physiologique (1 ml)
Groupe 2 :     héparine commerciale à 1600 U/kg
Groupe 3 :     héparine commerciale à 3200 U/kg
Groupe 4 :     héparine commerciale à 6400 U/kg

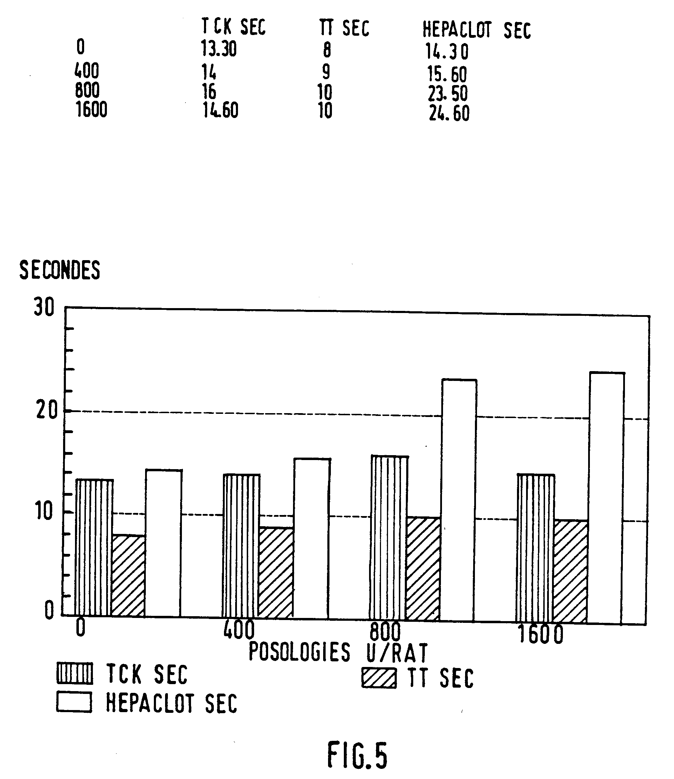

Les tests réalisés sont les tests TCK, T.T, HEPACLOT (STAGO) précédemment définis. Les résultats de ces tests sont rapportés à la figure 5.

Ces résultats confirment l'absorption d'héparine par voie orale, notamment à des doses variant entre 400 et 800 U/kg.

## EXEMPLE II

### Administration par voie orale d'une composition contenant de l'héparine, et des proportions variables de chlorure de calcium à des volumes variables.

Les concentrations relatives en ose et en sel ionique, utilisées en combinaison avec l'héparine, ne sont pas sans importance. C'est ce que fait apparaître cet exemple.

Dix lapins mâles adultes, d'un poids moyen de 2 kg de race "Nouvelle Zélande" provenant de l'élevage EVIC CEBA, reçoivent par voie orale une solution de 1ml contenant au total 2500 unités d'héparine, soit 1250 unités d'héparine par kg de lapin, à laquelle ont été additionnés des volumes croissants, échelonnés entre 0,1 et 0,9 ml d'une solution de chlorure de calcium (solution à 10% soit 10g Ca/100 ml).

Le résultat correspondant à un volume de 0,5ml de solution de chlorure de calcium n'a pas été retenu, parce qu'erroné en raison d'une erreur de manipulation. La composition administrée est par ailleurs dépourvue de glucose.

Les lapins sont anesthésiés avec l'anesthésique connu sous la marque commerciale BRIETAL (30 mg/kg). (Laboratoire PARKE-DAVIS). Des prélèvements sont opérés immédiatement (TO) et respectivement, 1 heure (1 H), 2 heures (2 H) et 3 heures (3 H) plus tard.

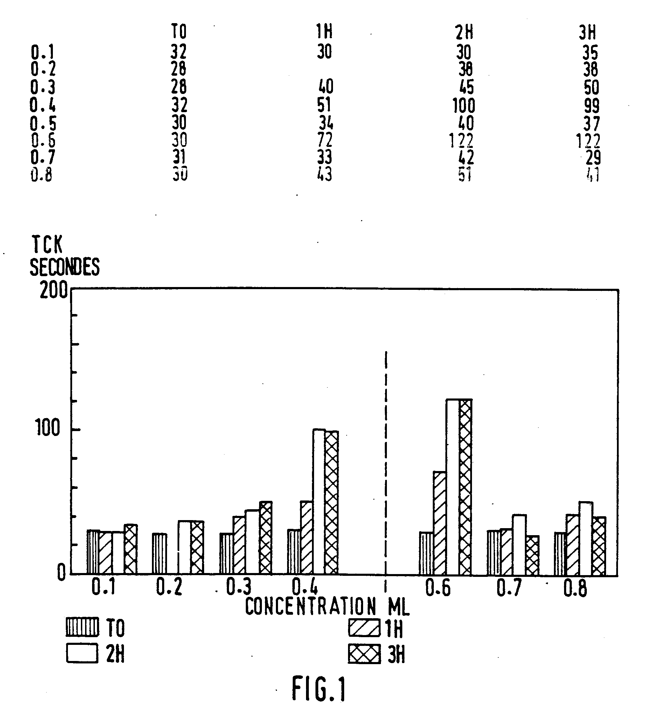

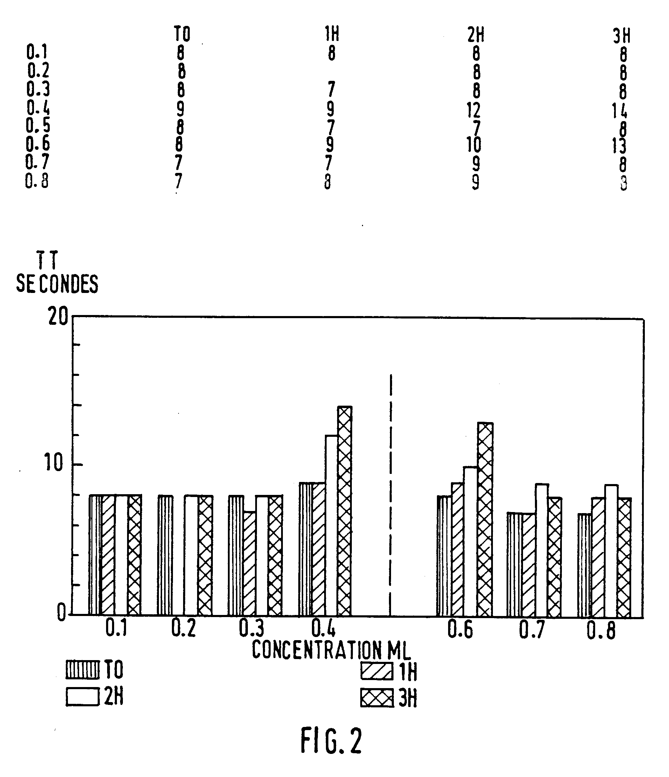

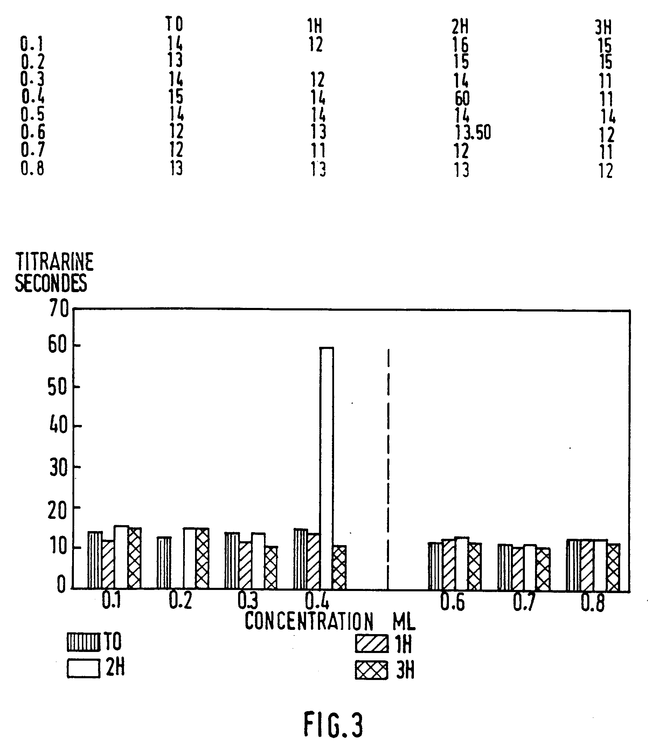

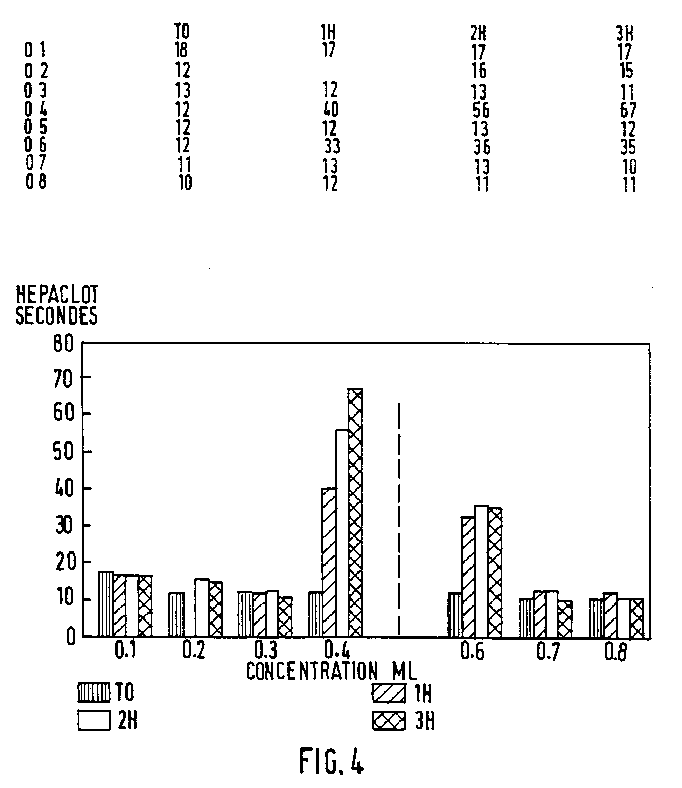

Les résultats des mesures des tests biologiques T.C.K., Titrarine, anti Xa, Hépaclot (STAGO) découlent des figures 1, 2, 3 et 4. Les résultats des tests permettent de déterminer un niveau d'efficacité supérieur, des compositions selon l'invention, lorsque le sel de calcium est administré en une quantité variant entre 0,3 ml et 0,6 ml de $CaCl_2$ par rapport au volume de solution d'héparine et d'héparine utilisée, soit lorsque le sel de calcium est utilisé à raison de 30mg à 60mg de chlorure de calcium, pour 2500 unités USP ou (2500 unités Anti-Xa ou 16mg d'héparine).

## EXEMPLE III

### Administration par voie orale d'une composition d'héparine à dose constante, associée à différents produits.

Les animaux utilisés sont des lapins mâles adultes de race "Néo-Zélandaise" d'un poids moyen de 2kg provenant de l'élevage EVIC CEBA et entretenus avec les aliments commercialisés sous la marque VIGALA des Grands Moulins de Paris.

Les constituants des mélanges de cet exemple sont :
- l'héparine sous forme d'héparinate de calcium, ayant une activité de 160 unités/mg (unités USP, ou anti-XA)
- soit du chlorure de sodium, soit du chlorure de calcium,
- du glucose ou du levulose.

L'héparine est administrée à raison de 1250 unités/kg de lapin dans un volume de 0,8ml.

Les mélanges A, B, C, D et E administrés par voie orale, sous forme de solutions, à des groupes distincts, qui comprenaient chacun 3 lapins, avaient les compositions découlant respectivement des tableaux qui suivent.

Les croix figurant dans chaque ligne du tableau III identifient les constituants du mélange correspondant (identifiés dans la partie supérieure du tableau). L'indication E.D. signifie que le produit administré est l'eau distillée. Les proportions relatives (en mM/l) des différents ions des constituants présents dans chacun de ces mélanges résultent du tableau IV.

TABLEAU III

|   | Hep.Ca + + | NaCl | CaCl$_2$ | Glucose | Levulose | E.D. |
|---|---|---|---|---|---|---|
| A | + | + |   | + |   |   |
| B | + |   | + | + |   |   |
| C | + |   | + |   | + |   |
| D | + |   | + |   |   | + |
| E | + |   |   |   |   | + |

TABLEAU IV

|   | A | B | C | D | E |
|---|---|---|---|---|---|
| Na + | 810 | 4 | 2 | 2 | 2 |
| K + | 3 | 1 | 0 | 0 | 0 |
| Ca + + | 50 | 273 | 271 | 267 | 73 |
| Chlorure | 920 | 1026 | 1020 | 1200 | 680 |
| GLUCOSE | 114 | 120 | 2,5 | 0 | 0 |

Les administrations ont été effectuées, après anesthésie au BRIETAL, injecté par voie intra-musculaire.

Les tests biologiques permettant le dosage plasmatique ont été effectués et les résultats sont donnés dans les tableaux suivants. Les tableaux fournissent des résultats pour chaque lapin de chaque groupe, et en secondes.

**T.C.K.**

```
----------------------------------------------l----------------------------------

Heure      Groupe A     Groupe B  Groupe C  Groupe D  Groupe E
prélève-   1   2   3    1   2   3  1   2   3  1   2   3  1   2   3
ment
--------------------------------------------------------------------------------

O            23  23  18   22  22  22  22  24  24  25  22  20  22  22  18
--------------------------------------------------------------------------------

1 h          23  28  28   28  28  29  28  31  26  25  30  23  21  21  22
--------------------------------------------------------------------------------

2 h          24  34  31   30  30  29  45  41  35  28  30  20  20  22  23
--------------------------------------------------------------------------------

3 h          24  27  27   27  25  24  35  48  40  26  29  20  20  22  20
--------------------------------------------------------------------------------
```

## T. T.

| Heure prélèvement | Groupe A | | | Groupe B | | | Groupe C | | | Groupe D | | | Groupe E | | |
|---|---|---|---|---|---|---|---|---|---|---|---|---|---|---|---|
| | 1 | 2 | 3 | 1 | 2 | 3 | 1 | 2 | 3 | 1 | 2 | 3 | 1 | 2 | 3 |
| O | 8 | 7 | 7 | 8 | 8 | 7 | 8 | 7 | 7 | 8 | 7 | 7 | 7 | 7 | 7 |
| 1 h | 9 | 8 | 10 | 8 | 8 | 9 | 9 | 8 | 9 | 8 | 8 | 8 | 7 | 7 | 7 |
| 2 h | 8 | 9 | 10 | 7 | 9 | 9 | 11 | 9 | 9 | 8 | 8 | 8 | 7 | 7 | 7 |
| 3 h | 8 | 9 | 9 | 7 | 8 | 9 | 9 | 9 | 9 | 8 | 7 | 7 | 7 | 7 | 7 |

## TITRARINE

| Heure prélèvement | Groupe A | | | Groupe B | | | Groupe C | | | Groupe D | | | Groupe E | | |
|---|---|---|---|---|---|---|---|---|---|---|---|---|---|---|---|
| | 1 | 2 | 3 | 1 | 2 | 3 | 1 | 2 | 3 | 1 | 2 | 3 | 1 | 2 | 3 |
| O | 12 | 12 | 12 | 11 | 12 | 11 | 12 | 12 | 12 | 11 | 12 | 11 | 12 | 12 | 12 |
| 1 h | 13.5 | 14 | 15 | 13 | 13 | 15 | 13 | 14 | 14 | 14 | 14 | 12 | 12 | 12 | 12 |
| 2 h | 13 | 13 | 15 | 14 | 16 | 15 | 19 | 15 | 15 | 13 | 14 | 12 | 12 | 12 | 12 |
| 3 h | 12 | 14 | 14 | 12 | 13 | 14 | 14 | 14 | 14 | 13 | 14 | 12 | 12 | 12 | 12 |

## HEPACLOT

| Heure prélève-ment | Groupe A | | | Groupe B | | | Groupe C | | | Groupe D | | | Groupe E | | |
|---|---|---|---|---|---|---|---|---|---|---|---|---|---|---|---|
| | 1 | 2 | 3 | 1 | 2 | 3 | 1 | 2 | 3 | 1 | 2 | 3 | 1 | 2 | 3 |
| O | 13 | 12 | 13 | 12 | 12 | 13 | 12 | 12 | 12 | 13 | 11 | 12 | 12 | 12 | 12 |
| 1 h | 14 | 18 | 17 | 14 | 16 | 14 | 19 | 15 | 12 | 14 | 13 | 12 | 12 | 12 | 13 |
| 2 h | 14 | 19 | 18 | 16 | 18 | 15 | 50 | 34 | 30 | 14 | 14 | 12 | 12 | 12 | 13 |
| 3 h | 14 | 26 | 16 | 16 | 14 | 14 | 42 | 41 | 40 | 14 | 12 | 12 | 12 | 12 | 13 |

OBSERVATIONS :

A la lecture des résultats précédents, on remarque que l'héparinate de calcium seul n'est pas absorbé (groupe E des lapins) ce qui souligne l'intérêt de la composition selon l'invention.

Le glucose peut être remplacé avantageusement par du lévulose l'absorption étant supérieure d'après les résultats obtenus (groupes B et C). On précise que le lévulose ou fructose est un isomère du glucose, dont il se différencie uniquement par une fonction Cétone.

Les résultats obtenus après utilisation de chlorures associés à différents ions, semblent indiquer que la présence de l'ion chlorure est déterminante. Toutefois, le choix de l'ion associé n'est pas indifférent pour l'efficacité de la composition.

EXEMPLE IV

Administration d'héparine par voie orale chez un rat porteur d'une trombose veineuse expérimentale.

Pour cette expérience le protocole utilisé est le suivant :

Les rats sont anesthésiés par injection intra-péritonéale de BRIETAL à la dose de 60 mg/kg. Après ouverture de l'abdomen, la veine cave inférieure est isolée et ligaturée sous la veine rénale gauche (t = 0). L'abdomen est refermé sur les différents plans. Deux heures plus tard (to + 2) les solutions 1,2,3,4 et le Placebo sont administrés par voie orale et la solution 5 est administrée par voie sous-cutanée.

Afin de prélever le thrombus, six heures après l'induction de la stase l'abdomen de l'animal est réouvert et des ligatures sont posées sur les veines colatérales et sur la veine cave inférieure, 2 cm en amont de la première ligature. Le caillot est extrait, et porté à l'étuve 24 heures à 37° C. Le caillot sec est pesé. Du sang est prélevé par ponction intra-cardiaque.

L'administration a été faite selon un protocole dit de "randomisation" avec une table dite de "randomisation": administration en double aveugle.

5 groupes de rats ont été formés, qui correspondent à l'administration de 5 solutions différentes, obtenues à partir des produits suivants :
- héparine, sel calcique
- glucosé 5%
- chlorure de calcium 10%
- bicarbonate de sodium.

La composition des solutions est donnée dans le tableau ci-après :

| | Calciparine ml | CaCL$_2$ ml | Glucose ml | ED ml | Bicarbonate de sodium ml |
|---|---|---|---|---|---|
| sol.1 | 0,3 | 1,2 | 0,9 | | |
| sol.2 | 0,3 | 1,2 | | 0,9 | |
| sol.3 | 0,3 | 1,2 | | | 0,9 |
| sol.4 | 0,3 | | 0,9 | | 1,2 |
| sol.5 | 0,3 | | | 2,1 | |

Pour chaque groupe de 10 rats la dose d'héparine administrée est de 1250 U./kg suivant la posologie indiquée ci-après :

Groupe 1 :　　0,10ml de la solution 1 par voie orale
Groupe 2 :　　0,10ml de la solution 2 par voie orale
Groupe 3 :　　0,10ml de la solution 3 par voie orale
Groupe 4 :　　0,12ml de la solution 4 par voie orale
Groupe 5 :　　0,10ml de la solution 5 par voie sous-cutanée
Groupe 6 :　　0,10ml de serum physiologique (Placebo)

Chaque groupe comprend dix rats.

L'administration des solutions a été faite par voie orale, 2 heures après induction d'une thrombose (ligature V.C.I. précitée). les solutions 1 à 4 sont administrées par voie orale, ainsi que le Placebo (groupe 6). La solution 5 est administrée par voie sous cutanée. Les poids moyens des thrombi ont alors été mesurés et ils sont répertoriés ci-après.

Pois moyens des thrombi en mg :

Groupe 1 :　　1,26 ± 1,01
Groupe 2 :　　1,89 ± 1,42
Groupe 3 :　　2,58 ± 1,08
Groupe 4 :　　2,64 ± 2,36
Groupe 5 :　　0,61 ± 0,59
Groupe 6 :　　2,50 ± 1,42

Une étude comparative des poids moyens des thrombi, menée en utilisant la formule statistique de Student, fait apparaître les résultats suivants :

1/ Etude comparative des poids moyens des thrombi des groupes 1 à 5 avec le groupe Placebo 6 :

| | |
|---|---|
| Groupe 1/ Groupe 6 | $p < 0,05$ |
| Groupe 2/ Groupe 6 | NS |
| Groupe 3/ Groupe 6 | NS |
| Groupe 4/ Groupe 6 | NS |
| Groupe 5/ Groupe 6 | $p < 0,05$ |

2/ Etudes comparative des poids moyens des thrombi des groupes 1 à 4 avec le groupe 5 :

| | |
|---|---|
| Groupe 1/ Groupe 5 | NS |
| Groupe 2/ Groupe 5 | $p < 0,05$ |
| Groupe 3/ Groupe 5 | $p < 0,05$ |
| Groupe 4/ Groupe 5 | $p < 0,05$ |

OBSERVATIONS :

Ces tests confirment donc la nécessité de produire une composition contenant au moins un mucopolysaccharide héparinique, un ose et un sel ionique selon l'invention.

En effet, d'après les résultats statistiques relatifs à l'étude comparative des poids moyens des thrombi, seuls les groupes 1 et 5 présentent une différence avec le groupe placebo 6. Ceci atteste donc en faveur de l'activité d'une composition à 3 composants selon l'invention, injectée par voie orale.

Par ailleurs les résultats comparatifs mettant en oeuvre d'une part les solutions testées par voie orale, d'autre part, la solution injectée par voie sous-cutanée confirment l'intérêt de la composition administrée par voie orale puisque son activité n'apparaît pas différente de celle de la solution administrée par voie sous-cutanée.

L'administration, chez le rat porteur d'une thrombose veineuse expérimentale et dans des conditions semblables, d'Héparine par voie orale conduit à un effet antithrombotique identique à celui obtenu avec de l'Héparine administrée par voie sous-cutanée.

Enfin, l'adjonction de bicarbonate de sodium semble provoquer une alkalose du milieu qui diminue ou annule l'activité de la composition selon l'invention sur la thrombose.

Enfin des études anatomopathologiques réalisées sur des animaux du type de ceux utilisés dans les expériences précédentes, ne révèlent aucune lésion de la muqueuse gastrique et duodénale, aux doses utilisées dans les exemples précédents.

**Revendications**

1. Composition caractérisée en ce qu'elle contient en combinaison :
   a/ au moins un composé mucopolysaccharide héparinique,
   b/ au moins un ose, physiologiquement acceptable, capable d'être absorbé au niveau de la muqueuse intestinale,
   c/ un sel ionique physiologiquement acceptable d'un cation métallique, les constituants a/, b/ et c/ étant en proportions mutuelles permettant l'induction par ladite composition d'un effet anticoagulant in vivo lorsqu'elle est administrée par voie orale.

2. Composition selon la revendication 1, caractérisée en ce que l'ose est choisi parmi les hexoses,

3. Composition selon la revendication 2, caractérisée en ce que l'hexose est le glucose,

4. Composition selon la revendication 2, caractérisée en ce que l'hexose est le lévulose,

5. Composition selon l'une quelconque des revendications 1 à 4, caractérisée en ce que le sel du cation est un chlorure,

6. Composition selon l'une quelconque des revendications 1 à 5, caractérisée en ce que le sel du cation est un chlorure de cation alcalinoterreux,

7. Composition selon la revendication 6, caractérisée en ce que le sel de cation est le chlorure de calcium,

8. Composition selon l'une des revendications 1 à 5, caractérisée en ce que le sel de cation est un chlorure de cation alcalin,

9. Composition selon la revendication 8, caractérisée en ce que le sel de cation est le chdlorure de sodium,

10. Composition pharmaceutique pour l'administration par voie orale caractérisée en ce qu'elle comprend la composition selon la revendication 1 en une dose efficace pour induire un effet anticoagulant in vivo, en association avec un véhicule pharmaceutique acceptable, approprié pour l'administration par voie orale.

11. Composition pharmaceutique selon les revendications 1, 3, 6, 8, caractérisée en ce qu'elle contient:
    - de 600 à 2500 unités USP de mucopolysaccharide héparinique
    - de 4 à 60mg d'ose
    - de 0,5 à 100mg de sel ionique de ce cation
    en association avec un véhicule pharmaceutique acceptable.

12. Composition pharmaceutique selon les revendications 1, 3, 6, 8, caractérisée en ce qu'elle contient :
    - de 600 à 2500 unités anti-Xa de mucopolysaccharides hépariniques,
    - de 4 à 60mg d'ose
    - de 0,5 à 100mg de sel ionique de ce cation
    en association avec un véhicule pharmaceutique acceptable,

**13.** Composition pharmaceutique selon les revendications 1, 3, 6, 8, caractérisée en ce qu'elle contient :
- de 4 à 17mg de mucopolysaccharide héparinique
- de 4 à 60mg d'ose
- de 0,5 à 100mg de sel ionique de ce cation

en association avec un véhicule pharmaceutique acceptable,

**14.** Composition pharmaceutique selon une quelconque des revendications 11 à 13, caractérisée en ce qu'elle contient :
- de 600 à 2500 unités USP de mucopolysaccharide héparinique
- de 4 à 50mg de glucose
- de 10 à 80mg de chlorure de calcium

en association avec un véhicule pharmaceutique acceptable.

**15.** Composition pharmaceutique selon l'une quelconque des revendications 11 à 13, caractérisée en ce qu'elle contient :
- de 600 à 2500 unités anti-Xa de mucopolysaccharide héparinique
- de 4 à 50mg de glucose
- de 10 à 80mg de chlorure de calcium

en association avec un véhicule pharmaceutique acceptable.

**16.** Composition pharmaceutique selon l'une quelconque des revendications 11 à 13, caractérisée en ce qu'elle contient :
- de 4 à 17mg de mucopolysaccharide héparinique,
- de 4 à 50mg de glucose
- de 10 à 80mg de chlorure de sodium

en association avec un véhicule pharmaceutique acceptable.

**17.** Composition pharmaceutique selon l'une quelconque des revendications 11 à 13, caractérisée en ce qu'elle contient :
- de 600 à 2500 unités USP ou de 600 à 2500 unités anti-Xa ou de 4 à 17mg de mucopolysaccharide héparinique
- de 4mg à 60mg de lévulose
- de 10 à 80mg de chlorure de calcium

en association avec un véhicule pharmaceutique acceptable.

**18.** Composition pharmaceutique selon l'une quelconque des revendications 11 à 13, caractérisée en ce qu'elle contient :
- de 600 à 2500 unités anti-Xa de mucopolysaccharide héparinique,
- de 4mg à 60mg de lévulose
- de 10 à 80mg de chlorure de calcium

en association avec un véhicule pharmaceutique acceptable.

**19.** Composition pharmaceutique selon, l'une quelconque des revendications 11 à 13, caractérisée en ce qu'elle contient :
- de 4mg à 17mg de mucopolysaccharide héparinique,
- de 4mg à 60mg de lévulose
- de 10 à 80 mg de chlorure de calcium

en association avec un véhicule pharmaceutique acceptable.

**20.** Composition pharmaceutique selon l'une quelconque des revendications 11 à 13, caractérisée en ce qu'elle contient :
- de 600 à 2500 unités USP de mucopolysaccharide héparinique
- de 5 à 50mg de glucose
- de 0,9 à 9mg de chlorure de sodium

en association avec un véhicule pharmaceutique acceptable.

**21.** Composition pharmaceutique selon l'une quelconque des revendications 11 à 13, caractérisée en ce qu'elle contient :

- de 600 à 2500 unités anti-Xa de mucopolysaccharide héparinique,
- de 5 à 50mg de glucose
- de 0,9 à 9mg de chlorure de sodium

22. Composition pharmaceutique selon l'une quelconque des revendications 11 à 13, caractérisée en ce qu'elle contient :
    - de 4 à 17mg de mucopolysaccharide héparinique
    - de 5 à 50mg de glucose
    - de 0,9 à 9mg de chlorure de sodium
en association avec un véhicule pharmaceutique acceptable.

23. Composition pharmaceutique selon l'une quelconque des revendications 1 à 22, destinée à être utilisée comme médicament, caractérisée en ce qu'elle est susceptible d'être administrée à un patient selon une dose contenant de 1000 à 100.000 unités anti-Xa d'héparine et de façon répétée 3 à 4 fois par jour.

24. Application d'une composition selon l'une quelconque des revendications 1 à 23 à la production de médicament administrable par voie orale ayant une activité régulatrice, in vivo vis-à-vis de la coagulation sanguine.

**Claims**

1. A composition containing, in combination :
   a) at least one heparinic mucopolysaccharide compound,
   b) at least one physiologically acceptable ose capable of being absorbed in the intestinal mucosa.
   c) a physiologically acceptable ionic salt of a metallic cation, the constituents a), b) and c) being in mutual proportions permitting induction, by the said composition, of an anticoagulant effect in vivo when it is administered orally.

2. The composition as claimed in claim 1, wherein the ose is chosen from among the hexoses.

3. The composition as claimed in claim 2, wherein the hexose is glucose.

4. The composition as claimed in claim 2, wherein the hexose is levulose.

5. The composition as claimed in any one of claims 1 to 4, wherein the cation salt is a chloride.

6. The composition as claimed in any one of claims 1 to 5, wherein the cation salt is an alkaline-earth cation chloride.

7. The composition as claimed in claim 6, wherein the cation salt is calcium chloride.

8. The composition as claimed in any one of claims 1 to 5, wherein the cation salt is an alkaline cation chloride.

9. The composition as claimed in claim 8, wherein the cation salt is sodium chloride.

10. A pharmaceutical composition for oral administration, comprising the composition as claimed in claim 1 at a dose capable of inducing an anticoagulant effect in vivo, in combination with an acceptable pharmaceutical vehicle suitable for oral administration.

11. The pharmaceutical composition as claimed in claims 1, 3, 6, 8, containing:
    - from 600 to 2,500 USP units of heparinic mucopolysaccharide
    - from 4 to 60 mg of ose
    - from 0.5 to 100 mg of ionic salt of this cation
    in combination with an acceptable pharmaceutical vehicle.

12. The pharmaceutical composition as claimed in claims 1, 3, 6, 8, containing:

15

- from 600 to 2,500 anti-Xa units of heparinic mucopolysaccharides,
- from 4 to 60 mg of ose
- from 0.5 to 100 mg of ionic salt of this cation

in combination with an acceptable pharmaceutical vehicle.

13. The pharmaceutical composition as claimed in claims 1, 3, 6, 8, containing:
- from 4 to 17 mg of heparinic mucopolysaccharide
- from 4 to 60 mg of ose
- from 0.5 to 100 mg of ionic salt of this cation

in combination with an acceptable pharmaceutical vehicle.

14. The pharmaceutical composition as claimed in any one of claims 11 to 13, containing:
- from 600 to 2,500 USP units of heparinic mucopolysaccharide
- from 4 to 50 mg of glucose
- from 10 to 80 mg of calcium chloride

in combination with an acceptable pharmaceutical vehicle.

15. The pharmaceutical composition as claimed in any one of claims 11 to 13, containing:
- from 600 to 2,500 anti-Xa units of heparinic mucopolysaccharide
- from 4 to 50 mg of glucose
- from 10 to 80 mg of calcium chloride

in combination with an acceptable pharmaceutical vehicle.

16. The pharmaceutical composition as claimed in any one of claims 11 to 13, containing:
- from 4 to 17 mg of heparinic mucopolysaccharide,
- from 4 to 50 mg of glucose
- from 10 to 80 mg of sodium chloride

in combination with an acceptable pharmaceutical vehicle.

17. The pharmaceutical composition as claimed in any one of claims 11 to 13, containing:
- from 600 to 2,500 USP units or from 600 to 2,500 anti-Xa units or from 4 to 17 mg of heparinic mucopolysaccharide
- from 4 mg to 60 mg of levulose
- from 10 to 80 mg of calcium chloride

in combination with an acceptable pharmaceutical vehicle.

18. The pharmaceutical composition as claimed in any one of claims 11 to 13, containing:
- from 600 to 2,500 anti-Xa units of heparinic mucopolysaccharide,
- from 4 mg to 60 mg of levulose
- from 10 to 80 mg of calcium chloride

in combination with an acceptable pharmaceutical vehicle.

19. The pharmaceutical composition as claimed in any one of claims 11 to 13, containing:
- from 4 mg to 17 mg of heparinic mucopolysaccharide,
- from 4 mg to 60 mg of levulose
- from 10 to 80 mg of calcium chloride

in combination with an acceptable pharmaceutical vehicle.

20. The pharmaceutical composition as claimed in any one of claims 11 to 13, containing:
- from 600 to 2,500 USP units of heparinic mucopolysaccharide
- from 5 to 50 mg of glucose
- from 0.9 to 9 mg of sodium chloride

in combination with an acceptable pharmaceutical vehicle.

21. The pharmaceutical composition as claimed in any one of claims 11 to 13, containing:
- from 600 to 2,500 anti-Xa units of heparinic mucopolysaccharide,
- from 5 to 50 mg of glucose
- from 0.9 to 9 mg of sodium chloride.

22. The pharmaceutical composition as claimed in any one of claims 11 to 13, containing:
    - from 4 to 17 mg of heparinic mucopolysaccharide
    - from 5 to 50 mg of glucose
    - from 0.9 to 9 mg of sodium chloride
    in combination with an acceptable pharmaceutical vehicle.

23. The pharmaceutical composition as claimed in any one of claims 1 to 22, intended to be used as a medicament, capable of being administered to a patient at a dose containing from 1,000 to 100,000 anti-Xa units of heparin, in a repeated manner 3 to 4 times per day.

24. Use of the composition as claimed in any one of claims 1 to 23 in the production of a medicament which can be administered orally and has a regulating activity in vivo vis-à-vis the blood coagulation.

**Patentansprüche**

1. Zusammensetzung, dadurch gekennzeichnet, daß sie in Kombination enthält:
    a) wenigstens eine heparinische Mucopolysaccharid-Verbindung,
    b) wenigstens eine physiologisch annehmbare Ose, die im Bereich der Intestinalmucosa absorbiert werden kann,
    c) ein physiologisch annehmbares ionisches Salz eines metallischen Kations, wobei die Bestandteile a), b) und c) in Anteilen vorgesehen sind, die in-vivo wechselseitig eine Antikoagulanswirkung der Zusammensetzung hervorrufen, wenn diese auf oralem Wege verabreicht wird.

2. Zusammensetzung gemäß Anspruch 1, dadurch gekennzeichnet, daß die Ose aus den Hexosen ausgewählt wird.

3. Zusammensetzung gemäß Anspruch 2, dadurch gekennzeichnet, daß die Hexose die Gluxose ist.

4. Zusammensetzung gemäß Anspruch 2, dadurch gekennzeichnet, daß die Hexose die Lävulose ist.

5. Zusammensetzung gemäß einem der Ansprüche 1 bis 4, dadurch gekennzeichnet, daß das Kationsalz ein Chlorid ist.

6. Zusammensetzung gemäß einem der Ansprüche 1 bis 5 dadurch gekennzeichnet, daß das Kationsalz ein Chlorid eines Erdalkalikations ist.

7. Zusammensetzung gemäß Anspruch 6, dadurch gekennzeichnet, daß das Kationsalz das Chlorid des Calciums ist.

8. Zusammensetzung gemäß einem der Ansprüche 1 bis 5, dadurch gekennzeichnet, daß das Kationsalz ein Chlorid eines Alkalimetallkations ist.

9. Zusammensetzung gemäß Anspruch 8, dadurch gekennzeichnet, daß das Kationsalz das Chlorid des Natriums ist.

10. Pharmazeutische Zusammensetzung zur oralen Verabreichung, dadurch gekennzeichnet, daß es eine Zusammensetzung gemaß Anspruch 1 in einer wirksamen Dosis umfaßt, um eine Antikoagulanzwirkung in-vivo hevorzurufen, in Kombination mit einem pharmazeutisch annehmbaren Träger, der zur oralen Verabreichung geeignet ist.

11. Pharmazeutische Zusammensetzung gemäß den Ansprüchen 1, 3, 6, 8, dadurch gekennzeichnet, daß sie enthält:
    - 600 bis 2500 USP-Einheiten des heparinischen Mucopolysaccharids,
    - 4 bis 60 mg Ose,
    - 0,5 bis 100 mg des ionischen Salzes des Kations,
    zusammen mit einem pharmazeutisch annehmbaren Träger.

17

**12.** Pharmazeutische Zusammensetzung gemäß den Ansprüchen 1, 3, 6, 8, dadurch gekennzeichnet, daß sie enthält:
- 600 bis 2500 Einheiten Anti-$X_a$ des heparinischen Mucopolysaccharids,
- 4 bis 60 mg Ose,
- 0,5 bis 100 mg des ionischen Salzes des Kations,

zusammen mit einem pharmazeutisch annehmbaren Träger.

**13.** Pharmazeutische Zusammensetzung gemäß den Ansprüchen 1, 3, 6, 8, dadurch gekennzeichnet, daß sie enthält:
- 4 bis 17 mg des heparinischen Mucopolysaccharids,
- 4 bis 60 mg Ose,
- 0,5 bis 100 mg des ionischen Salzes des Kations,

zusammen mit einem pharmazeutisch annehmbaren Träger.

**14.** Pharmazeutische Zusammensetzung gemäß einem der Ansprüche 11 bis 13, dadurch gekennzeichnet, daß sie enthält:
- 600 bis 2500 USP-Einheiten des heparinischen Mucopolysaccharids,
- 4 bis 50 mg Glucose,
- 10 bis 80 mg Calciumchlorid,

zusammen mit einem pharmazeutisch annehmbaren Träger.

**15.** Pharmazeutische Zusammensetzung gemäß einem der Ansprüche 11 bis 13, dadurch gekennzeichnet, daß sie enthält:
- 500 bis 2500 Einheiten Anti-$X_a$ des heparinischen Mucopolysaccharids,
- 4 bis 50 mg Glucose,
- 10 bis 80 mg Calciumchlorid,

zusammen mit einem pharmazeutisch annehmbaren Träger.

**16.** Pharmazeutische Zusammensetzung gemäß einem der Ansprüche 11 bis 13, dadurch gekennzeichnet, daß sie enthält:
- 4 bis 17 mg des heparinischen Mucopolysaccharids,
- 4 bis 50 mg Glucose,
- 10 bis 80 mg Natriumchlorid,

zusammen mit einem pharmazeutisch annehmbaren Träger.

**17.** Pharmazeutische Zusammensetzung gemäß einem der Ansprüche 11 bis 13, dadurch gekennzeichnet, daß sie enthält:
- 600 bis 2500 USP-Einheiten oder 600 bis 2500 Einheiten Anti-$X_a$ oder 4 bis 17 mg heparinisches Mucopolysaccharid,
- 4 mg bis 60 mg Lävulose,
- 10 bis 80 mg Calciumchlorid,

zusammen mit einem pharmazeutisch annehmbaren Träger.

**18.** Pharmazeutische Zusammensetzung gemäß einem der Ansprüche 11 bis 13, dadurch gekennzeichnet, daß sie enthält:
- 600 bis 2500 Einheiten Anti-$X_a$ heparinisches Mucopolysaccharid,
- 4 mg bis 60 mg Lävulose,
- 10 bis 80 mg Calciumchlorid,

zusammen mit einem pharmazeutisch annehmbaren Träger.

**19.** Pharmazeutische Zusammensetzung gemäß einem der Ansprüche 11 bis 13, dadurch gekennzeichnet, daß sie enthält:
- 4 mg bis 17 mg heparinisches Mucopolysaccharid,
- 4 mg bis 60 mg Lävulose,
- 10 bis 80 mg Calciumchlorid,

zusammen mit einem pharmazeutisch annehmbaren Träger.

**20.** Pharmazeutische Zusammensetzung gemäß einem der Ansprüche 11 bis 13, dadurch gekennzeichnet, daß sie enthält:
- 600 bis 2500 USP-Einheiten heparinisches Mucopolysaccharid,
- 5 bis 50 mg Glucose,
- 0,9 bis 9 mg Natriumchlorid,

zusammen mit einem pharmazeutisch annehmbaren Träger.

**21.** Pharmazeutische Zusammensetzung gemäß einem der Ansprüche 11 bis 13, dadurch gekennzeichnet, daß sie enthält:
- 600 bis 2500 Einheiten Anti-$X_a$ heparinisches Mucopolysaccharid,
- 5 bis 50 mg Glucose,
- 0,9 bis 9 mg Natriumchlorid,

zusammen mit einem pharmazeutisch annehmbaren Träger.

**22.** Pharmazeutische Zusammensetzung gemäß einem der Ansprüche 11 bis 13, dadurch gekennzeichnet, daß sie enthält:
- 4 bis 17 mg heparinisches Mucopolysaccharid,
- 5 bis 50 mg Glucose,
- 0,9 bis 9 mg Natriumchlorid,

zusammen mit einem pharmazeutisch annehmbaren Träger.

**23.** Pharmazeutische Zusammensetzung gemäß einem der Ansprüche 1 bis 22, dazu bestimmt als Medikament eingesetzt zu werden, dadurch gekennzeichnet, daß sie geeignet ist einem Patienten mit einer Dosis, die 1000 bis 100.000 Einheiten Anti-$X_a$ des Heparins in einer wiederholten Weise drei- bis viermal pro Tag verabreicht zu werden.

**24.** Verwendung einer Zusammensetzung gemäß einem der Ansprüche 1 bis 23 zur Herstellung eines Medikaments, das auf oralem Wege verabreichbar ist und das eine regulierende Wirkung in-vivo gegenüber der sanuinischen Koagulation hat.

|      | TO | 1H | 2H  | 3H  |
|------|----|----|-----|-----|
| 0.1  | 32 | 30 | 30  | 35  |
| 0.2  | 28 |    | 38  | 38  |
| 0.3  | 28 | 40 | 45  | 50  |
| 0.4  | 32 | 51 | 100 | 99  |
| 0.5  | 30 | 34 | 40  | 37  |
| 0.6  | 30 | 72 | 122 | 122 |
| 0.7  | 31 | 33 | 42  | 29  |
| 0.8  | 30 | 43 | 51  | 41  |

FIG.1

|      | TO | 1H | 2H | 3H |
|------|----|----|----|----|
| 0.1  | 8  | 8  | 8  | 8  |
| 0.2  | 8  |    | 8  | 8  |
| 0.3  | 8  | 7  | 8  | 8  |
| 0.4  | 9  | 9  | 12 | 14 |
| 0.5  | 8  | 7  | 7  | 8  |
| 0.6  | 8  | 9  | 10 | 13 |
| 0.7  | 7  | 7  | 9  | 8  |
| 0.8  | 7  | 8  | 9  | 3  |

T T
SE CONDES

CONCENTRATION ML

TO    1H
2H    3H

FIG. 2

|      | T 0 | 1H    | 2H    | 3H |
|------|-----|-------|-------|----|
| 0.1  | 14  | 12    | 16    | 15 |
| 0.2  | 13  |       | 15    | 15 |
| 0.3  | 14  | 12    | 14    | 11 |
| 0.4  | 15  | 14    | 60    | 11 |
| 0.5  | 14  | 14    | 14    | 14 |
| 0.6  | 12  | 13    | 13.50 | 12 |
| 0.7  | 12  | 11    | 12    | 11 |
| 0.8  | 13  | 13    | 13    | 12 |

FIG.3

|      | TO | 1H | 2H | 3H |
|------|----|----|----|----|
| 0 1  | 18 | 17 | 17 | 17 |
| 0 2  | 12 |    | 16 | 15 |
| 0 3  | 13 | 12 | 13 | 11 |
| 0 4  | 12 | 40 | 56 | 67 |
| 0 5  | 12 | 12 | 13 | 12 |
| 0 6  | 12 | 33 | 36 | 35 |
| 0 7  | 11 | 13 | 13 | 10 |
| 0 8  | 10 | 12 | 11 | 11 |

HEPACLOT
SECONDES

CONCENTRATION ML

▥ TO  ▨ 1H
☐ 2H  ▩ 3H

FIG. 4

| | T CK SEC | TT SEC | HEPACLOT SEC |
|------|----------|--------|--------------|
| 0 | 13.30 | 8 | 14.30 |
| 400 | 14 | 9 | 15.60 |
| 800 | 16 | 10 | 23.50 |
| 1600 | 14.60 | 10 | 24.60 |

SECONDES

POSOLOGIES U/RAT

▥ TCK SEC          ▨ TT SEC
☐ HEPACLOT SEC

FIG.5

24